# EUROPEAN PATENT APPLICATION

(11) **EP 1 728 560 A1**
(43) Date of publication of application: **06.12.2006**
(21) Application number: 05012016.1
(22) Date of filing: 03.06.2005
(51) Int. Cl.: B05C 17/005, A61C 5/06, A61M 5/19

(54) **System for storing and dispensing of a substance**

(71) Applicant: 3M Innovative Properties Company, St. Paul, Minnesota 55133-3427 (US)
(72) Inventor: Peuker, Marc, 86938 Schondorf (DE); Hohmann, Arno, 81369 München (DE); Pauser, Helmut, 86911 Diessen (DE)
(74) Representative: Vossius & Partner

(57) **Abstract**

The present invention is directed to a system for storing and dispensing a substance and a method for air free filling of the same. The system comprises at least one compartment for storing a predetermined amount of a substance, wherein said compartment has a back end and a front end for filling and dispensing the substance, respectively. The system further comprises a vent, a movable front plug and a displaceable piston for sealing the compartment at the back end, wherein the compartment defines a predetermined volume between the vent and the movable front plug within the compartment downstream of said piston.

## Description

### Field of the Invention

The present invention is directed to a storing and dispensing system for substances, preferably flowable substances, and a method which enables air free filling and storing a precise predetermined amount of substance. The storing and dispensing system of the present invention further provides a self-opening feature providing extended convenience in handling of the dispensing system.

### Background of the Invention

Standard syringe configurations are generally used as dose dispensing or delivering systems. The standard syringe configurations are either filled by a user or pre-filled with the desired substance. The manual filling of a standard syringe has the drawback that a precise filling volume depends on the user's handling skills, which due to the variability in skills leads to variability of volume. When the syringe is pre-filled, the filling tolerance is typically dependent on the filling device, wherein devices with a more precise filling are more expensive. For small containers of a rather low filling volume, as are used in the dental field, a precise filling of the required volume is essential especially for two- or multi-component compositions wherein a precise mixing ratio of the components is essential for the quality of the mixture.

A further drawback of standard pre-filled syringe configurations is the difficulty to provide encapsulation of the substance without voids or air bubbles. A precise filled volume of a substance with a reduced amount of voids or bubbles would for instance inject less air into the patients blood stream, in case the syringe is used for pharmaceutical substances. Furthermore, precise filled volumes for two- or multi-component compositions with a reduced amount of voids or bubbles in the mixture would enhance the strength and durability of the mixed composition.

US-A-6 261094 is directed to a capsule construction for dispensing an ultra dense composite dental restorative material by a syringe wherein the capsules include a body portion defining a reservoir and a connected nozzle angularly disposed relative to the axis of the body portion. The body portion defining a reservoir has an internal diameter which is equal to or only slightly larger than the internal diameter of the nozzle and the discharge orifice. A sealing cap is provided at one end of the capsule and a bi-directional piston is provided at the other end for extruding the ultra dense material from the capsule. The internal surfaces of the capsules may be coated with lubricating liquid compatible with the ultra dense composite material to be dispensed thereby.

US-A-3 603 310 relates to disposable dispensing syringes having a liquid-filled compartment which is separate from contact with a syringe cannula. The liquid-filled compartment is provided between a piercable inner piston and an outer piston. In storage the inner piston is partly pierced by the inner end of a two-way syringe cannula. The inner piston is axially movable from the sealing position to a dispensing position in which the inner end of the cannula pierces the inner piston and permits the liquid to be dispensed from the compartment.

Typical dispensing systems do not have a system for storing and later mixing material components and, therefore, may be inconvenient when dispensing substances which are made of material components that have to be mixed just before use. In this case, the material components first have to be mixed and then filled into the dispenser system, which can lead to the presence of undesirable voids and bubbles.

Therefore, there is a need for an improved storing and dispensing system and method offering improved bubble-free filling and storage of an exact amount of substance with minimal effort.

### Summary of the Invention

According to a first aspect, the present invention provides a system for storing and dispensing a substance, comprising at least one compartment for storing a substance, wherein the compartment has a back end and a front end for filling and dispensing the substance, respectively. The compartment further comprises a vent, preferably in the vicinity of the back end, and a movable front plug within the compartment downstream of the vent. The compartment is closeable with a displaceable rear plug or piston at the back end, wherein the compartment defines a predetermined volume between the vent and the movable front plug.

According to a second aspect, the present invention provides a system for storing and dispensing a substance comprising at least one compartment with a front end and a back end, a front plug and a displaceable rear piston. The front plug and the back piston are adapted to be spaced apart in the compartment to define a volume within the compartment, and are further adapted to be axially or longitudinally movable within the compartment. The compartment further comprises a vent preferably in the vicinity of the back end, wherein the volume of the chamber between the vent and the front plug in its initial position define a predetermined volume.

According to a third aspect, the present invention provides a system for storing and dispensing a substance comprising at least one compartment with a front end and a back end, a front plug and a displaceable rear piston. The front plug and the piston are adapted to be spaced apart in the compartment to define a volume within the compartment, and are further adapted to be axially movable within the compartment. The compartment further comprises an annular vent groove in the vicinity of the back end, wherein the volume in the chamber between the vent groove and the front plug placed in an initial position between the vent groove and the front end define a predetermined volume.

The vent of the present invention is preferably formed as an annular groove near the back end of the compartment. The vent may also be formed as a longitudinal groove, i.e., substantially parallel to the longitudinal axis of the compartment. Alternatively, the vent may be formed as a tapered portion at the back end of the compartment. The vent may also be formed as a widened portion and/or stepped configuration at the back end with at least one step.

The fill volume of the chamber may be filled with any material, e.g. with liquid, viscous or highly viscous substances, with pharmaceutical substances, dental fluid pharmaceuticals (such as enzymatic caries removal solutions), pastes, or any other substance(s) which is or are preferably stored and dispensed without interfering gas bubbles.

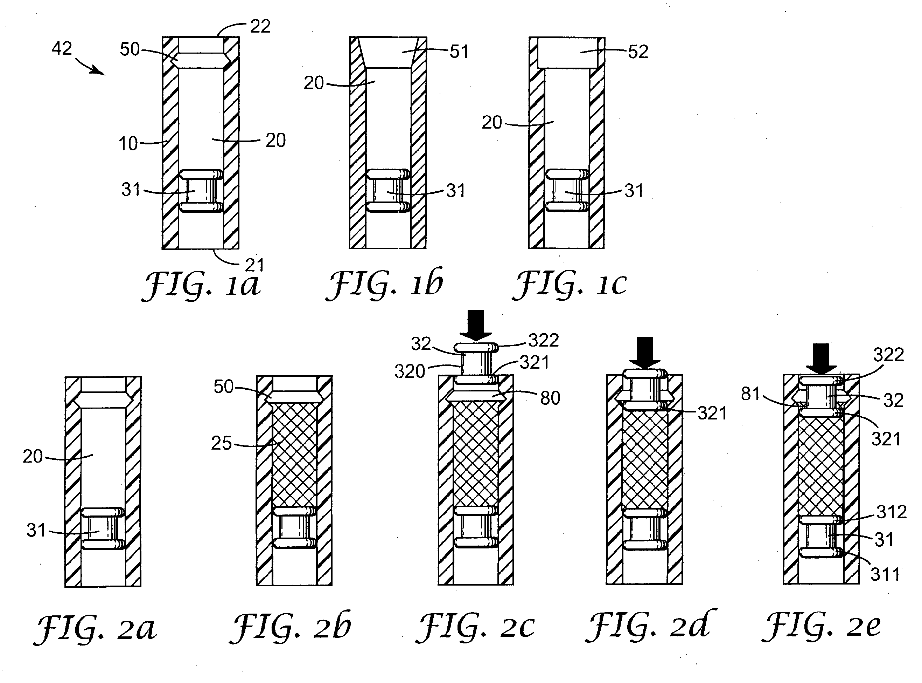

The front plug is preferably cylindrical having a circular cross-section. However, other cross-sectional shapes of the plug (e.g., square, polygonal, etc.) that match the shape of the compartment are also encompassed by the present invention. Preferably, the front plug comprises at least one sealing bulge. Even more preferred, the plug comprises at least first and second sealing bulges next to a front end and a back end of the plug, respectively, such as the plug shown in Fig. 1a. It is also preferred that the piston comprises at least first and second sealing bulges next to a front end and a back end of the piston, respectively. The plug and/or the piston may comprise a central cylindrical body portion having similar or identical disc-shaped end portions. Preferably the end portion of the rear piston facing the interior of the compartment chamber is of a shape that assists in getting air out of the compartment chamber when the piston is inserted. In particular, the end portion may be convex and hemispherical, ellipsoidal or of any other curvilinear or arcuate shape. The plug and piston may have a similar or equal shape and also both end portions of each the plug and the piston may be of a similar or equal shape. The first and second sealing bulges of the piston are spaced from each other such that any excess amount of the substance (beyond the predetermined volume) filled in the volume can at least in part be accommodated within the space defined between the two bulges, the central body of the piston and the compartment. Thus, any substance exceeding the predetermined volume may be retained between the first and second sealing bulges of the piston. The central body and the two bulges of the piston form with the wall of the compartment an annular space for accommodating such excess substance.

Preferably, the front plug is slightly harder to move than the rear piston so that if the rear piston is pushed to remove air and excess material the front plug does not move until the air and excess material is escaped. For example, the compartment is slightly tapered towards the front end so that a slightly higher friction is provided at the front end. In this case, the front plug and the rear piston can have the same diameter. Alternatively, the diameter of the front plug is slightly larger than the diameter of the rear piston. As a further alternative, the front plug and the rear piston are made from different materials (e.g. materials having different Shore Hardnesses).

The system of the present invention may be provided with a self-opening feature, such that for instance a fluid communication pathway between a compartment and an orifice is provided when the movable front plug is moved from its initial position towards the front end of the compartment.

According to another aspect, the present invention provides a system for storing and dispensing at least two substances. Additionally, the system may also assist the mixing of at least two substances. According to this aspect, the system comprises at least two separate compartments, wherein each compartment comprises a plug and a piston as explained above. The system further comprises an outlet for mixing and dispensing the substances, wherein each compartment comprises an orifice providing a fluid communication pathway between the respective compartments and the outlet. When the plugs are moved from their initial position towards the front end of the compartments and the plugs have passed the orifices, a fluid communication pathway between each compartment and the outlet is created enabling the substances of both compartments to flow towards the outlet. Preferably the compartments and the outlet are integrally formed as one piece. The system may further comprise a mixer such a static mixer in the outlet. Alternatively, the two compartments can comprise individual outlets in case an immediate mixing of the substances is not desired.

The system of the present invention preferably comprises a plunger for each compartment for applying pressure to the piston. Preferably but not necessarily, the plunger and the pistons are integrally formed as one piece.

Moreover, the invention is directed to a method of gas- or air-free filling and sealing a system for storing an exact amount of substances in a predetermined fill volume. The method comprises the steps of i) providing a compartment with a plug in an initial position inside the compartment; ii) filling the compartment from the back end with a substance at least up to a vent; wherein the initial position of the plug and the vent defines a predetermined volume; iii) placing a piston into the back end of the compartment; iv) further advancing the piston until the predetermined volume is air-free sealed between the piston and the plug. Preferably, between step iii) and step iv) the remaining air between the piston and the fill level of the substance is compressed.

The excess of substance filled above the vent is able to escape into or through the vent such that between the piston and the plug the predetermined volume of substance is enclosed. Depending on the design of the vent, the excess of substance is either removable from the system or maintained in the system. Preferably the excess of substance is sealed between the first and second sealing bulges of the piston as described above.

Further, the invention is directed to a method of mixing at least two substances with a storing and dispensing system according to the present invention with the steps of: (a) displacing the piston(s) towards the front end of the compartments and simultaneously displacing the plugs by hydraulic transmission of force via the substance stored in the compartment; (b) opening orifice when the plugs approach the front end of their respective compartments thus providing fluid communication between the compartments and the outlet; further advancing the pistons thus extruding the substances from the compartments into the outlet.

The present invention is advantageous in that the system enables air-or gas-free filling of a storing and dispensing system for substances without the need of complicated or expensive machinery or processes. The present invention is furthermore advantageous in that it provides a precise predetermined volume in the compartment even when used with filling devices with relatively large filling tolerances. Thus, the present invention is also advantageous in providing a precise volume of substances for small containers of a rather low filling volume as are used in the dental field. The present invention is also advantageous in that it provides a precise volume of substances without the high costs associated with precise filling machinery.

The present invention is further advantageous in that it provides a self-opening feature which means that no separate operation or step is necessary to open the syringe (e. g. remove cap, assemble tip etc.) before use. The syringe will automatically open when the plunger is advanced to a predetermined point, and provides precisely-controllable dispensing of a substance.

The present invention is further advantageous in that it provides a precise mixing ratio of the components especially for two- or multi-component compositions. The present invention is advantageous in that material components for substances can be pre-filled substantially without voids or air bubbles and stored in the dispensing system and then mixed automatically within the system prior to or during use. Also, since the delivery system of the present invention is disposable, there is no effort needed to clean the system after use.

### Brief Description of the Drawings

Further advantages will be apparent from the following description and drawings of the preferred embodiments of the present invention:
- Fig. 1a: is a cross-sectional view of a cartridge with a vent according to a first embodiment of the present invention;
- Fig. 1b: is a cross-sectional view of a cartridge with a vent according to a second embodiment of the present invention;
- Fig. 1c: is a cross-sectional view of a cartridge with a vent according to a third embodiment of the present invention;
- Figs. 2a to 2e: are cross-sectional views of a cartridge at different filling stages illustrating an air free filling of the cartridge with a vent according to a first embodiment of the present invention;
- Figs. 3a and 3b: are partly enlarged cross-sectional views according to Figs. 2d and 2e, respectively;
- Figs. 4a and 4b: are cross-sectional views of a cartridge with a vent according to the first embodiment of the present invention showing different plug positions during storage of the substance;
- Fig. 4c: is a cross-sectional view of a cartridge with a vent according to a fourth embodiment of the present invention;
- Figs. 5a to 5d: are cross-sectional views of a dispensing system with two compartments at different filling stages illustrating an air free filling of the cartridge with vents according an embodiment of the present invention;
- Fig. 6: is a partly enlarged cross-sectional view of the cartridge system according to Figs. 5a to 5d with an additional optional widened area between the groove and the compartment;
- Fig. 7a and 7b: are cross-sectional views of a dispensing system with two compartments at advancing stages of the plungers with a vent according an embodiment of the present invention;
- Fig. 8: shows a perspective sectional view of the front end of the cartridge system shown in Figs. 5a to 5d; and
- Figs. 9a to 9c: are cross-sectional views of a dispensing system with one compartment at different dispensing stages with a vent according an embodiment of the present invention.

### Detailed Description of Preferred Embodiments

In one preferred embodiment of the present invention as shown in Figs. 1a and Figs. 2a to 2e, the dispensing system 42 generally comprises a cartridge 10 having one compartment 20, e.g. in form of a chamber extending axially through the compartment for storing a substance. The compartment has a back end 22 and a front end 21 for filling and dispensing the substance, respectively. For sealing the compartment at the front end 21, a movable front plug 31 is inserted in the compartment 20, wherein the position of the front plug 31 in part defines the predetermined filling volume of the compartment as will be explained in further detail below.

The cartridge 10 according to the first embodiment of the invention furthermore comprises an annular vent groove 50 in the vicinity of the back end 22, which enables substantially air-free filling of the system. The vent groove eliminates or at least reduces that air is captured in the compartment. The cartridge is sealed at the back end 22 with a piston 32 (see Fig. 2c to 2e). The piston 32 of the preferred embodiment generally has a central cylindrical body portion 320 and comprises a first sealing bulge 321 and a second sealing bulge 322 at the front end and back end of the piston, respectively. Alternatively, the piston is shaped in the form of two truncated cones joined to each other at their respective peaks. The piston 32 may be designed symmetrically but other designs are also possible. The movable front plug 31 may have a design similar to the piston 32. Designs with only one sealing bulge are also encompassed. Preferably, the movable front plug 31 and the piston 32 may have an identical design and size such that production costs are reduced. The piston 32 and the plug 31 are designed to fit tightly and slideably into the compartment 20, and can have any desired cross-sectional shape (including square or rectangular shapes) provided that the compartment and the plug and piston have corresponding shapes. As can be seen in Fig. 2e, the piston 32 and the plug 31 have two bulges 321, 322 and 311, 312, respectively at their front ends and back ends forming a tight seal at the front and back end of the compartment 20. The piston 32 and plug 31 are preferably made of relative soft elastic material, for example rubber (EPDM, NBR etc.) or thermoplastic elastomers (e.g. TPE-U = Thermoplastic Polyurethanes, TPE-S = Styrene Block Copolymers, TPE-O = Thermoplastic Polyolefines, TPE-V = Thermoplastic Vulcanizate). The piston 32 can be integrally molded together with the bulges 321 and 322. Alternatively, or in addition, independently formed bulges can be attached to a plug (e. g. by using O-rings). Preferably, the bulges are made of a softer material appropriate to tightly seal the compartment.

The cartridge 10 of the dispensing system 42 has a body which is preferably made of relatively stiff material, for example polypropylene, polycarbonate, or polyoxymethylene. The body may be injection-molded.

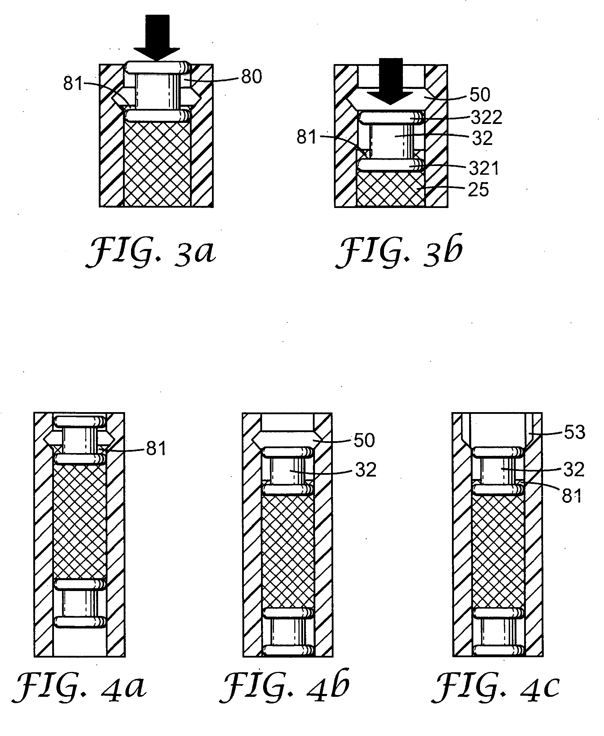

Fig. 1b shows a second embodiment of a vent, wherein the vent is designed as a tapered portion 51 at the back end of the compartment 20. Fig.1c shows a third embodiment of a vent, wherein the vent is designed as a stepped configuration 52 with one step at the back end of the compartment. Depending on the design of the vent, an excess of substance is either directly removable from the system (Fig. 1b and Fig. 1c) or (first) maintained in the system (see Fig. 1a, Fig. 3a and 3b). Preferably, the excess of substance is sealed between the first and second sealing bulges of the piston and the inner wall of the compartment.

Turning now to Figs. 2a to 2e, the filling process of a cartridge according to the present invention will be described in detail. As shown in Fig. 2a, a first plug 31 is placed into an initial position in the compartment 20 of the cartridge 10. The first plug 31, the cartridge 10, and the vent 50 form a compartment 20 with a predetermined volume, which is dependent on the initial position of the plug 31. As will be apparent from Fig. 2d, the predetermined volume is the volume within the compartment between the upper end of plug 31 and the lower edge of vent 50. In other words, the predetermined volume is achieved by an exact placement of the plug 31, because any additional material within the chamber that rises above the vent is excluded by the piston from the predetermined volume, assuming that no underfilling takes place. Thus, the tolerance of the predetermined volume is merely dependent on the tolerance of the cartridge and the positioning of the plug 31. In this context, it is to be noted that the front plug 31 can be placed more exactly (i.e., within a smaller tolerance) than a filling device can control the exact fill volume, especially a small fill volume below 1 ml. As shown in Fig. 2b, the compartment 20 is filled with substance 25, e.g. a dental material. The minimum fill level is the lower edge of the vent groove 50. Tolerances of the filling volume will result in higher fill levels. The fill volume of the filling device should be adjusted so that in every instance the chamber will be filled at least to the lower edge of the vent groove, so that the predetermined volume is in every instance completely full of material.

In the next step, as shown in Fig. 2c, the piston 32 is placed into the compartment thus closing the compartment. During insertion of the piston 32, the air 80 in the compartment is compressed until the first sealing bulge 321 of the piston 32 has reached the vent groove and the air can escape. As shown in Figs. 2d and 2e, the piston 32 is further advanced until it seals with the first sealing bulge 321 the compartment 20 at or below the lower edge of the vent groove 50, thus enclosing an air-free predetermined volume of substance between the piston 32 and plug 31. Within this arrangement, all excess material 81 above the lower edge of the vent groove 50 is separated from the other material and displaced to the annular space formed by the piston bulges 321, 322 and the compartment 20. In order to obtain a space to accommodate a sufficient amount of material, the piston 32 has a recessed area between the sealing bulges 321 and 322. Alternatively or additionally, sufficient space can be formed by the annular vent groove 50. The excess material 81 is tightly enclosed between the sealing bulges 321 and 322 of the piston and can remain in the dispensing system during storage and through eventual disposal. Figs. 3a and 3b are enlarged views which correspond to Figs. 2d and 2e.

Depending on the requirements of the compartment there are options for the "storage position" of the piston shown in Figs. 4a to 4c.

As an option instead of an annular groove, a longitudinal groove 53 can be used as shown in Fig. 4c. In this case compression of the air during plug insertion can be avoided. However, the annular groove provides the advantage that excess material cannot escape from the compartment after it has been sealed.

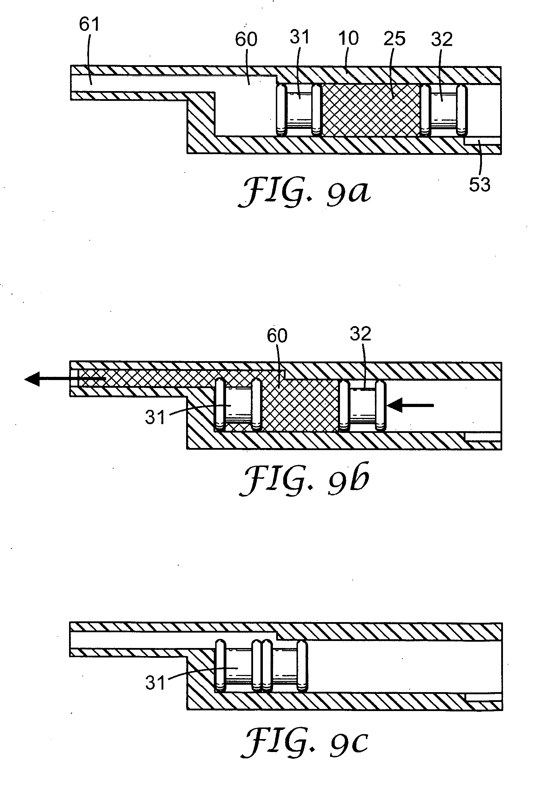

Turning now to Figs. 9a to 9c, the dispensing process of a cartridge with a single compartment according to the present invention will be described in detail. As shown in Fig. 9a, a first plug 31 is placed in an initial position in the compartment of the cartridge 10. The first plug 31 and the piston 32 seal a predetermined amount of substance 25 in the volume of the compartment. The present invention is advantageous in that it provides a self-opening feature as shown in Figs. 9b and 9c. "Self-opening" means that no separate operation or step is necessary to open the syringe (e. g. remove cap, assemble tip etc.) before use. Therefore, the syringe will automatically open when the piston 32 is advanced (indicated by the arrow in Fig. 9b) sufficiently to enable the material to exit the compartment, and provides controllable dispensing of a substance. Advancing the piston 32 towards the front end of the compartment transmits by hydraulic transmission, via the substance stored in the compartment, the advancing force to the plug 31. The displacement of the movable plug 31 toward the front end of the compartment provides a fluid communication path between the compartment and the outlet 61 of the cartridge. In other words, the displacement of the plug 31 to the front end allows the substance 25 to pass-by the plug 31 through the orifices 60 into the outlet 61. A further advancing of the piston 32 extrudes the substance from the compartment into the outlet 61.

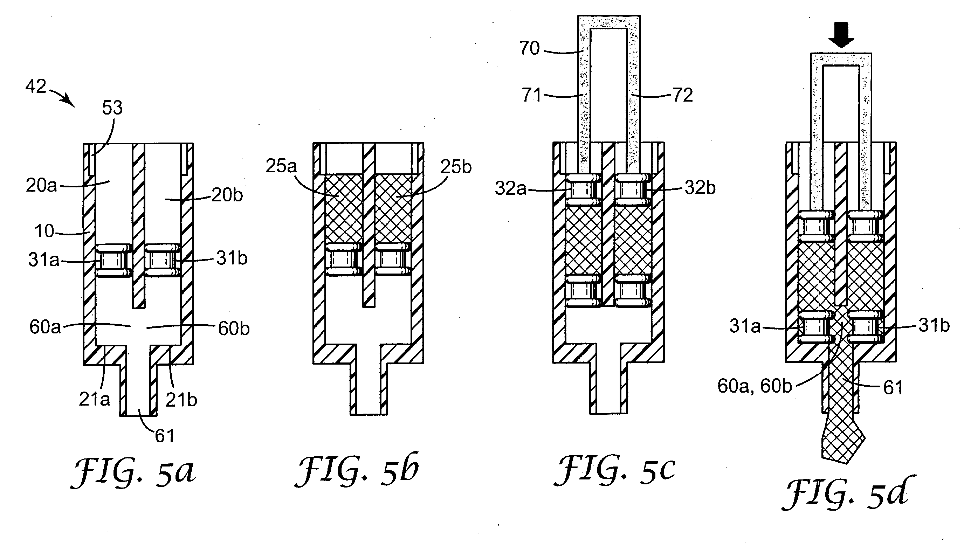

In another preferred embodiment of the present invention, as shown in Figs. 5 to 8, the dispensing system 42 generally comprises a cartridge 10 with two compartments 20a and 20b for storing two components or substances, an outlet 61 for mixing the substances, and a plunger 70 having rods 71, 72 associated with pistons 32a, 32b and corresponding to material compartments 20a, 20b for enabling controlled filling, storing and dispensing of the substances to be mixed. The compartments or chambers 20a and 20b provide space for storing substances or material components in separate areas within the dispensing system. This allows the system to be pre-filled and store material or substance components separately until use, which is useful for mixtures of materials that include a catalyst as one component. Although only two substance compartments are shown in Figs. 5 to 8, more than two material compartments containing different substance components may be provided, if desired. Each material compartment 20a, 20b is via orifices 60a and 60b in fluid communication with the outlet 61 of the cartridge 10 when actuated, and the material components are transferred into the outlet 61. The outlet 61 may comprise a static mixer for passively mixing the material components as they are forced out of the device.

The filling process for this embodiment is similar to the process as described above with respect to Figs. 2a to 2e. However, the compartments shown in Figs. 5a to 5d and 8 comprise a vent with at least one longitudinal groove 53, i.e. a groove in axial direction of the cartridge 10, rather than the annular groove 50 as described with respect to Figs. 2a to 2e. However, other vent shapes such as helical vent grooves can be used as an alternative, and more than one vent (as with the longitudinal or helical vents) can be provided if desired.

Preferably, the outlet 61 is designed as one channel being in fluid communication with both compartments 20a and 20b via the orifices 60a and 60b, thus allowing the substance components to flow together when they are extruded from the compartments simultaneously. However, alternatively, the cartridge comprises an outlet with two separate channels to keep the materials separate in a downstream step or to be used as single components.

Preferably the cartridge 10 with the two compartments 20a and 20b is integrally formed in a single piece. An optional separation wall 11 separates the two compartments 20a and 20b. The separation wall 11 has an aperture close to the front end of the cartridge 10, thus forming the orifices 60a and 60b and providing fluid communication between the two compartments 20a and 20b as well as with the outlet (see, e.g., Fig. 8).

As described above, the substances 25a, 25b are encapsulated between pistons 32a, 32b and the plugs 31a, 31b within the compartments 20a and 20b. To extrude the material from the cartridge, the pistons 32a, 32b are pushed forward. Since the material is preferably relatively incompressible this forces the front plugs 31a, 31b to be moved forward as well. As soon as the plugs 31a, 31b have passed the aperture or the orifices 60a and 60b, fluid communication between both compartments 20a and 20b and the outlet 61 is created. This enables the substances of both compartments to flow into the outlet 61. The outlet may contain a static mixer (not shown).

It has been observed that moving two pistons and two plugs at a time may require a rather high initial force, because the static forces necessary to begin moving each piston and plug from a resting position would all be applied at the same time. Depending on the material of the pistons and plugs and the cartridge as well as depending on the time the parts have been stored in assembled condition, this initiation force usually is considerably higher than the dynamic (sliding) friction of the plugs and pistons once they are in motion. This means that a solution is preferred that would enable easier initial movement of the pistons and plugs.

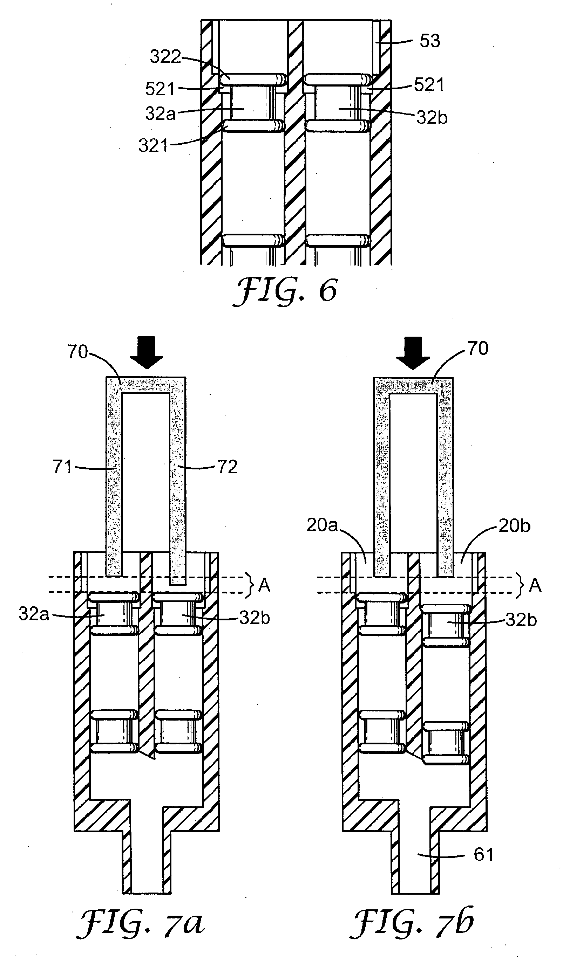

According to one embodiment, annular recesses 521 are integrated in the back end of the compartments, as shown in Fig. 6. Therefore, two of the eight sealing bulges of this embodiment are only slightly compressed at the recess portions, thus requiring lower initiation forces. This way the initiation force can be reduced by up to 25% of its value when both plugs and both pistons must be moved at once. Further embodiments may also include recesses at the front end of the compartments. For example, a stepped configuration as shown in Fig. 6 may also be provided at the front end of the compartments so that the sealing bulges 311 at the front end of the front plugs 31a, 31b are less compressed than the back end sealing bulges 312 of the front plugs 31a, 31b.

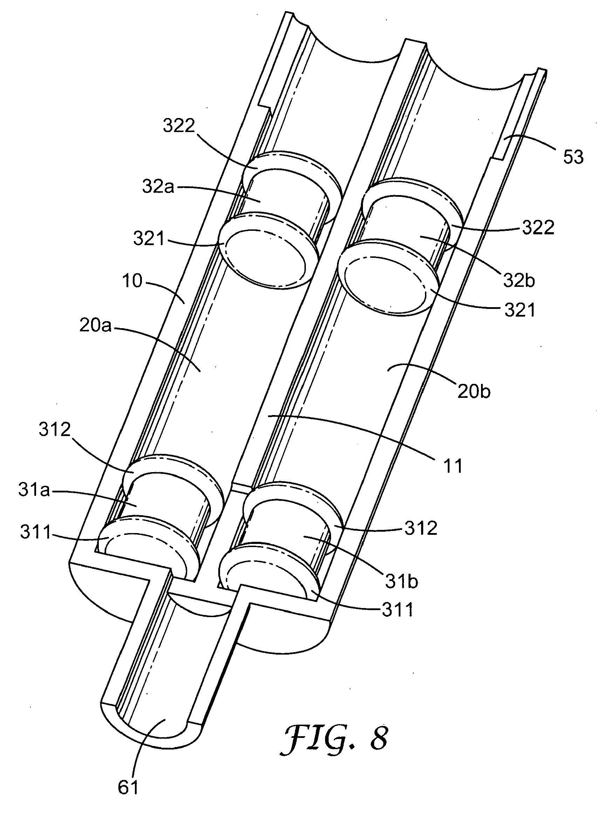

According to another embodiment, shown in Fig. 7a, the two rods 71 and 72 of the double plunger 70 have a different lengths. Note reference lines "A". In this way, only the piston 32b of one compartment is moved at the beginning of the dispensing process, which provides about 50% lower initiation force relative to moving the plugs of both compartments at one time. Another embodiment is shown in Fig. 7b, wherein the two rods 71 and 72 of the double piston have the same length and a different stepped configuration in the two compartments 20a and 20b is provided. In other words, the desired difference is provided by displaced pistons 32a and 32b. Preferably, these two alternatives are combined.

The present invention is not limited to the specific illustrated embodiments. Moreover, the present invention is realized by the features of the claims and any obvious modifications thereof.

### List of Reference signs

- 10: cartridge
- 11: separation wall
- 20: compartment
- 21: front end of compartment
- 22: back end of compartment
- 25: substance
- 31: front plug
- 32: rear piston
- 311: first sealing bulge of front plug
- 312: second sealing bulge of front plug
- 321: first sealing bulge of rear piston
- 322: second sealing bulge of rear piston
- 42: dispensing system
- 50: vent groove
- 51: tapered portion
- 52: stepped configuration
- 521: annular recess
- 53: longitudinal groove
- 60: orifice
- 61: outlet
- 70: plunger
- 71: rod
- 72: rod
- 80: air
- 81: excess material

## Claims

**1.** A system for storing and dispensing a substance, the system comprising:
at least one compartment for storing a substance, said compartment having a back end and a front end for filling and dispensing the substance, respectively;
a vent;
a movable front plug; and
a displaceable piston at the back end;
wherein the compartment defines a predetermined volume between the vent and the movable front plug within the compartment downstream of said displaceable piston.

**2.** A system for storing and dispensing substance, the system comprising:
at least one compartment with a front end and a back end;
a front plug and a displaceable piston being adapted to be spaced apart in the compartment to define a volume within the compartment, and being further adapted to be axially movable within the compartment; and
a vent in the vicinity of the back end;
wherein the vent and the front plug in its initial position define a predetermined volume.

**3.** A system for storing and dispensing a substance, the system comprising:
at least one compartment with a front end and a back end;
a front plug and a displaceable back plug being adapted to be spaced apart in the compartment to define a volume within the compartment and being further adapted to be axially movable within the compartment; and
an annular vent groove in the vicinity of the back end;
wherein the vent groove and the front plug placed in an initial position between the vent groove and the front end define a predetermined volume.

**4.** The system according to claim 1 or 2, said vent comprising an annular vent groove.

**5.** The system according to claim 1 or 2, said vent having a tapered portion at the back end of the compartment.

**6.** The system according to claim 1 or 2, said vent having a stepped configuration at the back end with at least one step.

**7.** The system according to any of the preceding claims, wherein said plug and/or said piston comprise at least first and second annular sealing bulges next to a front end and a back end of said plug and/or piston, respectively.

**8.** The system according to claim 7, wherein said first and second sealing bulges of said piston are spaced from each other to form a space such that excess substance in said volume is at least in part accommodateable within said space.

**9.** The system according to any of claims 1 to 8, wherein said predetermined volume is filled with a substance.

**10.** The system according claim 9, wherein said substance is a pharmaceutical substance.

**11.** The system according claim 10, wherein said pharmaceutical substance is a dental fluid pharmaceutical.

**12.** The system according to claim 11, wherein said dental fluid pharmaceutical is a enzymatic caries removal solution.

**13.** The system according to any of claims 9 to 12, wherein said substance exceeding the predetermined volume is sealed between the first and second sealing bulges of said piston.

**14.** The system according to any of the preceding claims, wherein a fluid communication pathway between the compartment and an orifice is provided when said plug is moved from its initial position a predetermined distance towards the front end of the compartment.

**15.** The system according to any of the preceding claims, wherein the system comprises two individual compartments and an outlet for mixing and dispensing said substances, wherein each compartment comprises an orifice providing a fluid communication pathway between said compartments and said outlet, when the plugs are moved from their initial position towards the front end of the compartments.

**16.** The system according to claim 15, wherein when the plugs are moved forward and the plugs have passed the orifice a fluid communication pathway between both compartments and the outlet is created enabling the substances of both compartments to flow to the outlet.

**17.** The system according to claim 15 or 16, wherein the compartments and the outlet are integrally formed as one piece.

**18.** The system according to any of the preceding claims, the system further comprising a static mixer in the outlet.

**19.** The system according to any of the preceding claims, wherein the system comprises a plunger for each compartment for applying pressure to the piston(s).

**20.** The system according to claim 19, wherein the plunger and the piston(s) are integrally formed as one piece.

**21.** A method of filling and sealing a storing and dispensing system, the method comprising the steps of:
i) providing a compartment with a plug in an initial position inside the compartment;
ii) filling the compartment from the back end with a substance at least up to a vent; wherein the initial position of the plug and the vent define a predetermined volume;
iii) placing a piston into the compartment at its back end; and
iv) further advancing the piston until the predetermined volume of the substance is sealed between the piston and the plug, wherein air is able to escape through the vent during at least a portion of the advancement.

**21.** The method according to claim 21, wherein between step iii) and step iv) the remaining air between the piston and the fill level of the substance is compressed.

**22.** The method according to claim 21 or 22, wherein between step iii) and step iv) the excess substance filled above the vent is able to escape through the vent such that between the piston and the plug the predetermined volume of substance is enclosed.

**23.** The method according to claim 21, 22 or 23, wherein excess substance is sealed between a first and second sealing bulge of the piston.

**24.** A method of mixing at least two substances by a storing and dispensing system according to any of claims 15 to 20, the method comprising the steps of:
i) displacing of the piston(s) towards the front end of the cartridge;
ii) simultaneously displacing the plugs by hydraulic transmission of force via the substance stored in the compartment;
iii) opening pathways when the plugs approach the front end of their compartment thus providing fluid communicating between the compartments and the outlet;
iv) further advancing the pistons thus extruding the substances from the compartments into the outlet.
